# EUROPEAN PATENT SPECIFICATION

(11) **EP 4 188 327 B1**
(45) Date of publication and mention of the grant of the patent: **28.05.2025**
(21) Application number: 21755917.8
(22) Date of filing: 30.07.2021
(51) Int. Cl.: A61K 9/00, A61K 31/573, A61K 47/10, A61P 11/08, A61K 31/439, A61K 31/58, A61K 31/56, A61K 31/167, A61K 31/40, A61K 45/06

(54) **COMBINATION THERAPY FOR INHALATION ADMINISTRATION**
KOMBINATIONSTHERAPIE ZUR INHALATIONSVERABREICHUNG
THÉRAPIE COMBINÉE POUR ADMINISTRATION PAR INHALATION

(30) Priority: 31.07.2020 EP 20382710
(43) Date of publication of application: 07.06.2023
(73) Proprietor: Chemo Research, S.L., 28050 Madrid (ES)
(72) Inventor: NIETO ORELLANA, Alejandro, 28806 Alcalá de Henares (ES); MAESO PUERTOLLANO, María, 28880 Meco (ES); ANDRADE BENITEZ, Laura, 28100 Madrid (ES)
(74) Representative: Aera A/S
(86) International application number: PCT/EP2021/071369
(87) International publication number: WO 2022/023515

(56) References cited:
- WO-A1-2017/089404
- WO-A1-97/01329
- US-A1- 2003 181 478

## Description

### Field of the invention

The present invention is related to a pharmaceutical composition for soft mist inhaler administration comprising a combination of two or three of an inhaled corticosteroid (ICS) with a long-acting β2-agonist (LABA) and a long-acting muscarinic antagonist (LAMA) to be used in the treatment of respiratory diseases, especially in asthma and chronic obstructive pulmonary disease (COPD), and process for the preparation thereof. The invention also relates to the use of said pharmaceutical formulation in a soft mist inhaler.

More particularly, the pharmaceutical compositions herein include the double therapy of beclometasone dipropionate (BPD) and formoterol fumarate (FF) and the triple therapy of beclometasone dipropionate (BPD), formoterol fumarate (FF) and glycopyrronium bromide (GB).

The compositions are propellant-free, multidose inhalation solutions intended for administration via soft mist inhaler.

### Background of the invention

The delivery of a drug by inhalation allows deposition of the drug in different sections of the respiratory tract, e.g., throat, trachea, bronchi and alveoli. Generally, the smaller the particle size, the longer the particle will remain suspended in air and the farther down the respiratory tract the drug can be delivered.

The use of dosage aerosols is well known as a strong part of the therapy of respiratory diseases, especially asthma and chronic obstructive pulmonary disease (COPD). Usually, metered dose inhalers are used by means of propellant gases. Following the recognition of the ozone damaging potential of these propellant gases, attempts to develop alternatives have increased. One alternative is the development of nebulizers, where solutions and suspensions of pharmacologically active substances are administered in the form of a mist into the lungs with the aid of a drug delivery device such as a nebulizer.

A nebulizer is a delivery device that was designed to overcome the pulmonary limitations of patients. Sometimes called a "breathing treatment," a nebulizer creates a mist containing the drug, which makes it easy and pleasant to breathe the drug into the lungs. A nebulizer requires formulations in liquid form to function properly. Nebulizers work by forcing air through a cup containing the liquid medicine. This produces tiny mist-like particles of the liquid so that they can be inhaled deeply into the airways. Other nebulizers use an ultrasonic mechanism to generate the mist.

The principle advantages of nebulizers over other methods of pulmonary installation are they completely disperse without the use of propellant gases; patient coordination between inhalation and actuation is not required and the delivery of higher doses of medication is easier. In the case of a soft mist inhaler, they are portable inhalers generating a mist without the use of external energy sources. This is one of the benefits of soft mist inhalers, but there are others compared to normal nebulizers. Soft mist inhalers deliver most of the mist to the lungs, with a very low amount of the drug being delivered to the mouth. Soft mist inhalers also deliver the full dose to the patient and none of it is wasted to the environment so this is also a safer approach.

A wide variety of nebulizers differing in mode of operation are available as for example the one described in PCT Patent Application WO 97/12687, a soft mist inhaler known under the trade name Respimat^{®}.

In the administration by nebulizers, usually the active ingredient is either suspended in micronized form in saline or dissolved in water-alcoholic mixtures in the presence of excipients such as buffering agents, stabilizing agents, surfactants and preservatives.

Thus, pharmaceuticals intended for inhalation by nebulization are dissolved or suspended in an aqueous or ethanolic solution, and according to the solution characteristics of the active substances, solvent mixtures of water and ethanol may also be suitable.

The maximum concentration of pharmaceutical in case of solutions is dependent on the solubility in the solvent and on the dosage required to achieve the desired therapeutic effect.

Propellant-free solution formulations of this kind are known in the art. Ethanolic formulations are disclosed for example in WO 97/01329, WO 2014/096115. Aqueous systems are described for example by WO 98/27959. Aqueous formulations for inhalation cannot be used, however, if the medicament ingredients are not sufficiently soluble in water. Starting from aqueous formulations, the solubility of formulation ingredients can, in some cases, be increased by adding ethanol to the aqueous system. However, it has been found that the ethanol concentration in an aqueous aerosol formulation critically influences the particle size distribution of the aerosol produced by nebulizer.

WO 02/083113 discloses a pharmaceutical composition comprising (i) formoterol, or a derivative thereof; and (ii) a steroidal anti-inflammatory agent, or a derivative thereof; in a pharmacologically suitable fluid, wherein the composition is stable during long term storage and the fluid comprises water and surfactants in order to dissolve the steroidal anti-inflammatory agent. The pharmaceutical compositions are suitable to be used in a nebulizer containing no propellant.

US 2007/293460 A1 and US 2007/098644 refer to a method of delivery of a combination therapy to the pulmonary system comprising: providing a nebulizer; providing an aqueous solution comprising a long-acting corticosteroid, a long-acting beta-agonist, and a long-acting anticholinergic; and administering said aqueous solution and surfactants in order to dissolve the long-acting corticosteroid to the patient using the nebulizer.

Propellant-free inhalable solutions are disclosed regarding any combination by using an aqueous and/or alcoholic solvent, preferably an ethanolic solution, in WO 2012/110462, wherein a muscarinic receptor antagonist, being dissolved in a solvent comprising at least 75% v/v of water and an optional co-solvent miscible with water; the aqueous solution may comprise 95% v/v of water and 5% v/v ethanol or 97.5% v/v water and 2.5% v/v ethanol.

WO 02/36106 discloses a pharmaceutical composition comprising anticholinergics and steroids, and WO 2006/114379 discloses a pharmaceutical composition comprising one or more anticholinergics, betamimetics and steroids, optionally in combination with pharmaceutically acceptable excipients. The solvent may be water on its own or a mixture of water and ethanol. The relative proportion of ethanol compared with water is limited to a maximum of 70 percent by volume.

WO 07/134968 discloses propellant-free, aqueous formulation for inhalation containing one or more active substances, optionally an excipient, and ethanol in an amount of from 10 to 50% (v/v).

US 2003/0181478 discloses formulations to be nebulized comprising tiotropium and Budesonide in a solution of a mixture of water (10 vol. %) and ethanol (90 vol. %) and additionally contain only benzalkonium chloride, an acid for adjusting the pH and sodium edetate.

WO 2015/193213 discloses a combination of a muscarinic antagonist, especially tiotropium, and a glucocorticoid such as ciclesonide formulated as an inhalation solution comprising either water alone, or a mixture of ≤95% V/V ethanol and ≥5% V/V water, such as 90% V/V ethanol and 10% V/V water, benzalkonium chloride, or stabilizers such as EDTA, butylhydroxyanisole or butylhydroxytoluene. Also, it is described that the formulations may further comprise a beta-2 adrenoceptor agonist, such as salbutamol (albuterol).

Preservative may be incorporated in inhaled formulations for nebulization to minimize the possibility of microbial contamination. The use of antimicrobial preservatives is less desirable, since certain of these have been associated with adverse clinical effects, such as pulmonary irritation, inflammation and bronchospasm. Alternative processes may be considered.

A combination of an inhaled corticosteroid (ICS) with a long-acting β2-agonist (LABA) and/or a long-acting muscarinic antagonist (LAMA) has been available for the treatment of asthma and chronic obstructive pulmonary disease (COPD). Particularly, the combination of beclometasone dipropionate (BPD), an inhaled corticosteroid, formoterol fumarate (FF), a long-acting beta-agonist, and glycopyrronium bromide (GB), a long-acting muscarinic antagonist is available under the brand name Trimbow^{®} (87mcg/5mcg/9mcg pressurized inhalation, solution) and the combination of beclometasone dipropionate (BPD), an inhaled corticosteroid, and formoterol fumarate (FF), a long-acting beta-agonist, is available under brand name Foster^{®} (100mcg/6mcg pressurized inhalation, solution), both by Chiesi Farmaceutici S.p.A. in a metered dose inhaler device.

Trimbow^{®} is a pressurised inhalation solution, also referred as pressurised metered dose inhaler (pMDI), containing three active substances (beclometasone dipropionate anhydrous, formoterol fumarate dihydrate and glycopyrronium bromide) dissolved in a medium consisting of norflurane (propellant), ethanol (co-solvent), hydrochloric acid (formulation stabiliser). Each delivered dose (the dose leaving the mouthpiece) contains 87 micrograms of beclometasone dipropionate, 5 micrograms of formoterol fumarate dihydrate and 9 micrograms of glycopyrronium (as 11 micrograms glycopyrronium bromide). Each nominal metered actuation (the dose leaving the valve) contains 100 µg BDP, 6 µg, FF and 10 µg glycopyrronium (as 12.5 micrograms glycopyrronium bromide).

MDI formulations of Beclometasone dipropionate (BDP) + Formoterol fumarate (FF) + Glycopyrronium Br (GB) are described in EP 2515853; EP 2515854; EP 2515855; EP 3089735; EP 3096737; EP 315181; EP 3500241.

Fostair^{®} 100/6 inhalation solution marketed by Chiesi Ltd., comprises 100 micrograms of beclometasone dipropionate, 6 micrograms of formoterol fumarate dehydrate, Norflurane (HFA-134a), Ethanol anhydrous and Hydrochloric acid to be used in a pressurised metered dose inhaler (pMDI). The product Fostair^{®} is described in EP 1787639.

WO 2017/089404 discloses an MDI formulation comprising formoterol fumarate, fluticasone propionate, and glycopyrronium bromide, as well as ethanol and HFA propellant.

Disadvantages of MDI formulations containing ICS alone or in combination with LABA and/or LAMA versus nebulization are the presence of propellant, lack of coordination between inhaler activation and patient inhalation (challenge for elderly and children), low lung deposition and high oropharyngeal deposition.

A method of improving the administration of drugs, such as inhaled corticosteroids alone or in combination with LABA and/or LAMA, by nebulization, particularly by soft mist inhaler, would be desired.

In view of the potential problems and disadvantages associated with the formulations containing inhaled corticosteroids alone or in combination with LABA and/or LAMA currently available on the market, it would be highly advantageous to provide formulations in solution, containing no propellant, stabilizing agents and/or preservatives, having an adequate shelf life, thus permitting an effective aerosol well tolerated by patients.

Therefore, there is a need to provide stabilized compositions of inhaled corticosteroid (ICS) with long-acting β2-agonist (LABA) and/or long-acting muscarinic antagonist (LAMA), free of propellant, stabilizing agents and/or preservatives.

Moreover, there is a need to improve the delivery of drug to the lungs, reducing the amount delivered to the mouth. Also, the full dose is delivered to the patient and none of it is wasted to the environment (safer approach). Additionally, the use of a soft mist inhaler is a portable system propellant free which provides better treatment efficiency (patient coordination between inhalation and actuation is not required).

### Summary of the invention

The present inventors have found a stable pharmaceutical composition for soft-mist inhaler administration comprising a combination of an inhaled corticosteroid (ICS) with a long-acting β2-agonist (LABA) and optionally a long-acting muscarinic antagonist (LAMA) to be used in the treatment of respiratory diseases, especially in asthma and chronic obstructive pulmonary disease (COPD). By being administered in the form of soft mist, wherein all the active ingredients are dissolved in an alcoholic solvent, higher lung deposition of the active ingredients is provided compared to other pharmaceutical forms. A solution of all components, including solubilised ICS, it is a more robust way of delivering drugs to the lungs, when compared to suspensions, as suspensions tend to sediment and it requires robust shaking of the particles before administering the drug.

Thus, in a first aspect the present invention relates to a pharmaceutical solution composition for delivery to the pulmonary system via a soft mist inhaler:
(a) an inhaled corticosteroid (ICS),
(b) a long-acting beta-agonist (LABA), and
(c) optionally a long-acting muscarinic antagonist (LAMA), dissolved in a pharmaceutically acceptable solvent, wherein the solvent comprises ethanol in an amount of 75 to 100% v/v.

More particularly, the pharmaceutical solution compositions are preferred wherein the inhaled corticosteroid is selected from the group consisting of beclomethasone dipropionate, budesonide, ciclesonide, fluticasone propionate, fluticasone furoate, and mometasone; the long-acting beta-agonist is selected from the group consisting of formoterol fumarate, salmeterol, indacaterol, vilanterol, and odolaterol; and the long-acting muscarinic antagonist is selected from the group consisting of glycopyrronium bromide, umeclidinium, aclidinium, ipratropium, tiotropium, and oxitropium.

The invention also relates to the use of said pharmaceutical formulation in a soft mist inhaler.

In a further aspect, the invention concerns the use of the present formulation in the manufacture of a medicament for the prevention and/or treatment of an inflammatory and/or obstructive airways disease, such as asthma or chronic obstructive pulmonary disease (COPD).

The principal advantage of the pharmaceutical formulations of the present invention is that they are completely dispersed without the use of propellant gases. In addition, they contain no stabilizing agents and/or preservatives, such as benzalkonium chloride (BAC) and disodium edetate.

Advantageously, the pharmaceutical composition of the present invention is stable over time.

Additionally, the pharmaceutical composition of the present invention offers improved solubility of the active ingredients providing stable solutions of active ingredients *versus* suspensions by using a simple process of preparation and less concentration of active ingredients in the formulation for an equivalent amount in the lung as soft mist inhalers are more efficient. Therefore, better aerodynamic performance than pMDIs (lower oropharyngeal deposition, higher deposition in the lungs) is obtained, wherein lower amounts of active ingredients are required to have similar therapeutic effect.

Moreover, due to the efficiency of the formulation and the nebulisation via a soft mist inhaler, such therapy of inhaled corticosteroids and beta-agonists and optionally long-acting muscarinic antagonists shows in-vitro an equivalent amount of drug delivered to the lungs from the inhaler and with an equivalent respirable fraction with 1 puff (actuation) when compared to 2 puffs (actuations) from p-MDI formulations. The formulation of the present invention delivered, with 1 puff, the same amount of drug to the lungs and with minor amount of drug being delivered to the oropharyngeal region compared to 2 puffs of the p-MDI formulations.

### Brief description of the figures

Figure 1: Aerodynamic size distribution of Formoterol. NGI, Formulation 1 (70% Ethanol) vs Formulation 2 (96% Ethanol).
Figure 2 : Aerodynamic particle size distribution of A) Beclometasone dipropionate, B) Glycopyrronium bromide and C) Formoterol Fumarate by NGI, Trimbow^{®} pMDI (2 actuations) vs Soft Mist Inhaler (1 actuation).
Figure 3: Aerodynamic particle size distribution of A) Beclometasone dipropionate and B) Formoterol Fumarate. NGI, Foster^{®} pMDI (2 actuations) vs Soft Mist Inhaler (1 actuation).
Figure 4: Aerodynamic size distribution of Budesonide. NGI, Formulation 1 (70% Ethanol) vs Formulation 2 (96% Ethanol).
Figure 5: Aerodynamic size distribution of Formoterol. NGI, Formulation 1 (70% Ethanol) vs Formulation 2 (96% Ethanol).
Figure 6: Aerodynamic size distribution of Budesonide. NGI, Symbicort^{®} pMDI (1 actuation) vs Soft Mist Inhaler (1 actuation).
Figure 7: Aerodynamic size distribution of Formoterol. NGI, Symbicort^{®} pMDI (1 actuation) vs Soft Mist Inhaler (1 actuation).

### Detailed description of the invention

### Definitions

All terms as used herein in this application, unless otherwise stated, shall be understood in their ordinary meaning as known in the art. Other more specific terms as used in the present application are as set forth below and are intended to apply uniformly throughout the specification and claims unless an otherwise expressly set out definition provides a broader definition.

Throughout the description and claims the word "comprise" and variations of the word, are not intended to exclude other technical features, additives, components, or steps. Furthermore, the word "comprise" encompasses the case of "consisting of". The following examples and drawings are provided by way of illustration, and they are not intended to be limiting of the present invention. Furthermore, the present invention covers all possible combinations of particular and preferred embodiments described herein.

The term "weight ratio by percentage" or "% w/w" refers to the percentage by weight of each active ingredient compared to the total weight of all active ingredients present in the pharmaceutical composition.

In the context of the present invention, the term "% v/v" refers to the percentage by volume of the solvent used in the formulation.

When referring to the term "preservative" in the context of the present invention, it refers to a substance effective in inhibiting microbial growth, such as growth of bacteria and fungi, in solutions, such as aqueous solutions. Examples include benzalkonium chloride (BAC) and various forms of edetate (ethylenediaminetetraacetate), such as disodium edetate.

In the context of the present invention, the term "propellant" refers to substances used to propel the active ingredients in metered dose inhalers, such as pMDIs. Typical propellants are hydrofluorocarbons, such as norflurane.

The expression "therapeutically effective amount" as used herein, refers to the amount of a compound that, when administered, is sufficient to prevent the development of, or alleviate to some extent, one or more of the symptoms of the disorder or condition being treated. The particular dose of compound administered according to this invention will of course be determined by the particular circumstances surrounding the case, including the particular condition being treated, the duration of the treatment, the nature of any concurrent treatment, and any other factor known to the expert.

The term "Nominal dose," as used herein, refers to the loaded dose, which is the amount of active pharmaceutical ingredient ("API") in an inhalation device prior to administration to the patient. The volume of solution containing the nominal dose is referred to as the "fill volume."

The "Fine Particle Fraction" (FPF) is defined as the percentage (%) of particles below 5 µm with respects to the delivered dose. It is also known as the "respirable fraction". This is defined in European Pharmacopoeia chapter 2.9.18 (Preparations for inhalation: Aerodynamic Assessment of Fine particles) and the equivalent United States Pharmacopoeial chapter is USP <601> Inhalation and Nasal Drug Products: Aerosols, Sprays and Powders- Performance Quality Tests.

In these chapters, the Fine Particle Dose (FPD) is defined by the mass of active substance less than 5 µm collected when inhalation products are actuated as per Pharmacopoeial chapters through any multistage impactor enabling aerodynamic particle size fractioning of the powder. The "aerodynamic particle size distribution" (APSD) is the product deposition in the multistage impactor obtained from the product actuation. The multiusage apparatus design defined in USP <601> consists of a connector between the impactor and the product (Induction Port), seven stages and a micro-orifice collector (MOC) or Internal Filter holder (IFH) for very fine formulations The Stages consist of removable cups and jets with multiple nozzles particle sizes for all stages except Stage 1, in decreasing order as the powder travels through the impactor. The sizes of these nozzles (µm) determine the aerodynamic deposition of the product powder in each of the stages and therefore enables the FPD calculation previously described.

The "delivered dose" (DD) is the amount delivered from the inhaler. This term is defined in European Pharmacopoeia chapter "Inhalanda: Preparations for inhalation" and for the USP in the previously mentioned chapter USP<601>. The delivered dose is obtained from the dose collection in a delivered dose apparatus when actuated at standard conditions defined in these chapters.

### Solution formulations

In one aspect, the present invention concerns a pharmaceutical solution composition for delivery to the pulmonary system via a soft mist inhaler comprising:
(a) an inhaled corticosteroid (ICS),
(b) a long-acting beta-agonist (LABA), and
(c) optionally a long-acting muscarinic antagonist (LAMA), dissolved in a pharmaceutically acceptable solvent, wherein the solvent comprises ethanol in an amount of 75 to 100% v/v. In one embodiment, the solvent comprises ethanol in an amount of 80-99% v/v. In another embodiment, the solvent comprises ethanol in an amount of 85-98% v/v. In a further embodiment, the solvent comprises ethanol in an amount of 90-97% v/v. In still another embodiment, the solvent comprises ethanol in an amount of 92-96% v/v. In yet another embodiment, the solvent comprises ethanol in an amount of 94-96% v/v. In still a further embodiment, the solvent comprises ethanol in an amount of approximately 96% v/v.

In one embodiment, the pharmaceutical solution composition comprises two active ingredients. In a further embodiment, said two active ingredients are an inhaled corticosteroid, such as beclomethasone dipropionate, and a long-acting beta-agonist, such as formoterol fumarate.

In one embodiment, the formulations according to the invention contain one or more pharmacologically acceptable acids and/or one or more buffers for adjusting the pH. In another embodiment, said acid is hydrochloric acid. In another embodiment, said buffer is citrate buffer.

In one embodiment, the formulations according to the invention do not contain a propellant. In a further embodiment, said propellant is a hydrofluorocarbon. In another embodiment, said propellant is norflurane.

The formulations of the present invention are prepared according to procedures well known in the art that comprise the mixing for some specific time the active ingredients with the solvent, such as ethanol, adjusting of the pH and further mixing for a specific time.

### Active ingredients

In one embodiment, the inhaled corticosteroid is selected from the group consisting of beclomethasone dipropionate, budesonide, ciclesonide, fluticasone propionate; fluticasone furoate, and mometasone; the long-acting beta-agonist is selected from the group consisting of formoterol fumarate, salmeterol, indacaterol, vilanterol, and odolaterol; and the long-acting muscarinic antagonist is selected from the group consisting of glycopyrronium bromide, umeclidinium, aclidinium, ipratropium, tiotropium, and oxitropium. In a further embodiment, the active substances that may be used in the formulations according to the invention are preferably selected from Beclomethasone dipropionate (BDP), Formoterol fumarate (FF), and optionally Glycopyrronium bromide (GB).

As used herein, Beclomethasone dipropionate (BDP) is a diester of beclomethasone (also referred to as beclometasone), a synthetic corticosteroid developed for the prophylactic management of mild, moderate, or severe asthma in adults or children and for the prophylactic treatment of chronic reversible obstructive airways disease. The chemical name is 9-chloro-11β,17,21-trihydroxy-16β-methylpregna-1,4-diene-3, 20-dione 17,21-dipropionate.

Formoterol is a selective β2-adrenergic receptor agonist. It is commercialized as formoterol fumarate (FF) and administered by oral inhalation. Formoterol acts locally in the lung as a bronchodilator. The chemical name of formoterol fumarate dihydrate is N-[2-Hydroxy-5-[(1RS)-1-hydroxy-2-[[(1RS-2-(4- methoxy-phenyl)-1-methylethyl] amino]ethyl]phenyl] formamide (E)-butenedioate dihydrate. Formoterol presents two asymmetric carbons, so four possible isomers exist, forming two racemates. The commercial formoterol is a racemic mixture of *R, R* (-) and *S, S* (+) enantiomers which is routinely ensured by a test for specific optical rotation. The content of diastereoisomers R*S* is controlled in the active substance.

Glycopyrrolate is a long-acting muscarinic antagonist. It is a synthetic quaternary amine known also as glycopyrronium. It is available in oral, intravenous and inhalation forms. Glycopyrrolate is a quaternary ammonium salt with the following chemical name: 3-(2-cyclopentyl-2-hydroxy-2-phenylacetoxy)-1,1-dimethylpyrrolidinium. The molecular formula is C₁₉H₂₈NO₃. There are two asymmetric carbons in the molecule. The product is a 50/50% mixture of the enantiomers. Thus, the product is not optically active. The counterion is typically bromide, in which case the long-acting muscarinic antagonist is glycopyrronium bromide (GB).

In one embodiment, the weight ratio by percentage (% w/w) of beclometasone dipropionate (BDP) to formoterol fumarate (FF) is generally 30-100% to 0.5-50%. In a further embodiment, the weight ratio by percentage is 50-100% to 0.5-25%, respectively. In another embodiment, the weight ratio by percentage is 70-100% to 0.5-10%, respectively. In still another embodiment, the weight ratio by percentage is 80-100% to 1-10%, respectively. In yet another embodiment, the weight ratio by percentage is 90-100% to 2-8%, respectively. In a further embodiment, the weight ratio by percentage is 94% to 6%.

In one embodiment, the weight ratio by percentage (% w/w/w) of the three active ingredients, beclometasone dipropionate (BDP), formoterol fumarate (FF), and glycopyrronium bromide (GB) is generally 30-100%, 0.5-50% and 0.5-50%, respectively. In a further embodiment the weight ratio by percentage is 50-100%, 0.5-25% and 1-25%, respectively. In another embodiment, the weight ratio by percentage is 70-100%, 0.5-10% and 2-20%, respectively. In still another embodiment, the weight ratio by percentage is 80-100%, 1-10% and 5-17%, respectively. In yet another embodiment, the weight ratio by percentage is 90-100%, 2-8% and 7-15%, respectively. In a further embodiment, the weight ratio by percentage is 84%, 11% and 5%, respectively.

### Nebulizers and soft mist inhalers

The nebulized formulations according to the invention have to meet high quality standards. The formulations according to the invention may be inhaled by oral or nasal route. Those soft mist inhalers which are capable of nebulizing a small amount of a liquid formulation in the dosage needed for therapeutic purposes within a few seconds into an aerosol suitable for therapeutic inhalation are particularly suitable. In one embodiment, the soft mist inhalers are those in which an amount of less than 25 microlitres, preferably less than 20 microlitres, most preferably less than 15 microlitres of active substance solution can be nebulized preferably in one puff to form an aerosol having an average particle size (or particle diameter) of less than 10 microns, preferably less than 5 microns, so that the inhalable part of the aerosol already corresponds to the therapeutically effective quantity.

As used herein, a "nebulized formulation" refers to a solution that is dispersed in the air to form an aerosol. Thus, a nebulized solution is a particular form of an aerosol. A nebulizer is an instrument that is capable of generating very fine liquid droplets for inhalation into the lung. Within this instrument, the nebulizing liquid or solution is atomized into a mist of droplets with a broad size distribution by methods known to those of skill in the art, including, but not limited to, compressed air, ultrasonic waves, or a vibrating orifice. Nebulizers may further contain, e.g., a baffle which, along with the housing of the instrument, selectively removes large droplets from the mist by impact. Soft mist inhalers are a specific type of nebuliser where the mist is produced by high pressure generated during device actuation, such as by releasing a coiled spring. These nebulizers do not need the aid of any external power source. Thus, the mist inhaled into the lung contains fine aerosol droplets. Nebulizers for use herein are soft mist inhalers.

In soft mist inhalers of this kind, the formulations in solutions are stored in a reservoir. It is important that the active substance formulations used are sufficiently stable when stored and at the same time are such that they can be administered directly, if possible, without any further handling, in accordance with their medical purpose. Moreover, they must not contain any ingredients which might interact with the inhaler in such a way as to damage the inhaler or the pharmaceutical quality of the solution or of the aerosol produced.

The nebulizer is a soft mist inhaler. The formulation according to the invention is nebulized using the technology of a soft mist inhaler, and the mass expelled, in at least 97%, preferably at least 98% of one actuation (puff), should correspond to a defined quantity with a range of tolerance of not more than 25%, preferably 20% of this quantity. Preferably, between 5-25 mg, more preferably between 10-15 mg of formulation are delivered as a defined mass per puff.

Preferably, the medicament combinations according to the invention are used as specified above for preparing a pharmaceutical composition for the treatment of obstructive pulmonary diseases selected from among bronchial asthma, severe asthma, acute asthma attacks, chronic bronchitis and chronic obstructive pulmonary disease (COPD), while it is particularly preferable according to the invention to use them for preparing a medicament for the treatment of bronchial asthma and COPD.

In view of the above description and the examples below, one of ordinary skill in the art will be able to practice the invention as claimed without undue experimentation. The foregoing will be better understood with reference to the following examples that detail certain procedures for the preparation of formulations according to the present invention.

The following examples should not be considered exhaustive, but merely illustrative of only a few of the many embodiments contemplated by the present invention.

### Examples

### Example 1 - Impact of ethanol in the solubilization and aerodynamic particle size distribution

A study was performed to evaluate the impact of ethanol in the solubilization of the components and the aerodynamic performance of an aerosol solution formulation of beclomethasone dipropionate (BDP), glycopyrronium bromide (GB) and formoterol fumarate (FF).

### Formulation

Beclometasone dipropionate (BDP), glycopyrronium bromide (GLB), formoterol fumarate (FF) and HCl 1N were mixed in 70% ethanol (Formulation 1) and 96% ethanol (Formulation 2) at 25°C for two hours protected from evaporation. Both formulations were filtrated, and individual assay of all active ingredients were tested by Reverse Phase HPLC.

**Table 1. Formulations investigated in this study.**

| **Solution** | **API/Excipients** | **Components composition** |
|---|---|---|
| Formulation 1 | Beclometasone Dipropionate | 87.4 µg |
| | Glycopyrronium Bromide | 10.9 µg |
| | Formoterol Fumarate | 5.2 µg |
| | HCl 1N | 2.5 µg |
| | Water for Irrigation | 3.32 µL |
| | Absolute Ethanol | 7.74 µL |
| Formulation 2 | Beclometasone Dipropionate | 87.4 µg |
| | Glycopyrronium Bromide | 10.9 µg |
| | Formoterol Fumarate | 5.2 µg |
| | HCl 1N | 2.5 µg |
| | Ethanol 96% | 11.05 µL |

### HPLC Analytical methods

Three independent Reverse Phase HPLC analytical methods were used for the determination of each active ingredient in all the analyses.

**Beclometasone Dipropionate:** An Isocratic (1.4mL/min), HPLC-UV (254nm) detection was employed in a 60/40 v/v organic (THF:AcN:MeOH 94:434:472 v/v/v)/ Aqueous (phosphate buffer pH 2.35) mobile phase elution in a C18 column (Kromasil 100 Å C18; 250x4.6mm; 5 µm). External Beclometasone Dipropionate standards prepared in the method diluent (56/44 v/v THF:AcN:MeOH 94:434:472 v/v/v/ phosphate buffer pH 2.35) were used to quantify the amount of active ingredient present in the sample prepared in the same diluent using the response factor of Standard and Sample solutions. Typical System Suitability Criteria applies as per USP <621> requirements.

**Glycopyrronium Bromide:** An Isocratic (1.0mL/min), HPLC-UV (225nm) detection was employed with a Mobile Phase composed of Sulphate (heptanosulphonate) buffer pH 5.9/MeOH:AcN:Sulphuric Acid 0.05M in a 61.5:15:23.5:0.3 v/v proportion with a C18 column (Zorbax Extend RR C18, 50 x 4.6 mm, 3.5 µm). External Glycopirronium Bromide standards prepared in the method diluent (35/65 v/v MeOH/Water) were used to quantify the amount of active ingredient present in the sample prepared in the same diluent using the response factor of Standard and Sample solutions. Typical System Suitability Criteria applies as per USP <621> requirements.

**Formoterol Fumarate:** An Isocratic (1.5mL/min), HPLC-Electrochemical detection was employed with a Mobile Phase composed of 76/24 v/v Phosphate Buffer pH 5.6: AcN v/v with a C18 column (Supelcosil LC-ABZ, 250 x 4.6 mm, 5 µm). External Formoterol Fumarate standards prepared in the method diluent (100 % MeOH) were used to quantify the amount of active ingredient present in the sample prepared in the same diluent using the response factor of Standard and Sample solutions. Typical System Suitability Criteria applies as per USP <621> requirements.

**Data analysis.** The Aerodynamic size distribution was plotted as a function of the stage cut-off diameter as the percentage of mass recovered from the induction port filter to the filter.

### Aerodynamic particle size distribution (APSD)

For the APSD evaluation Formulation 1 and 2 were filled into cartridges compatible with a soft mist inhaler (Respimat^{®}). The *in vitro* aerodynamic particle size distribution of both formulations was evaluated using an impactor, the next generation impactor, NGI (Copley Scientific Ltd) equipped with a mouthpiece adapter for the insertion of the inhaler, an induction port and an internal filter holder (IFH) to capture the smaller particles. The product was tested as per procedure detailed in the European pharmacopoeia chapter 2.9.18 (apparatus E) and US Pharmacopeia chapter <601> (apparatus 6) for Soft Mist Inhalers.

The NGI was cooled to 5°C for at least 75 min. The cooling chamber was then opened and, after 30 minutes, the NGI was connected to a HCP5 vacuum pump (Copley Scientific Ltd). For each experiment, ten doses were discharged per NGI actuated at 30 L/min for 5 seconds each.

After the required actuations, the particles deposited on the different surfaces of the impactor were extracted using a suitable diluent (methanol) and with the help of a Gentle Rocker (Copley Scientific Ltd). Active ingredients were then quantified by HPLC, with the same methods used for the assay testing described above This establishes the distribution of particles according to their aerodynamic particle size.

**Data analysis.** The Aerodynamic size distribution was plotted as a function of the stage cut-off diameter as the percentage of mass recovered from the induction port filter to the filter.

### Results

The assay of each active ingredient, summarized in the following Table 2, showed that the formulation providing the best results in terms of higher active ingredient content (in particular beclometasone dipropionate and formoterol fumarate) was Formulation 2 - formulation containing 96% ethanol. Without being bound by a particular theory, these results could be ascribed to the poor solubility of these compounds in water.

**Table 2. Active ingredient content in Formulation 1 and 2 after filtration. Formulation 1: 70% Ethanol; Formulation 2: 96% Ethanol.**

| **Active Pharmaceutical Ingredient** | **Theoretical concentration (mg mL⁻¹)** | **Formulation 1 Assay (%)⁽¹⁾** | **Formulation 2 Assay (%)⁽¹⁾** |
|---|---|---|---|
| Beclometasone Dipropionate | 7.9 | 40.1 | 99.7 |
| Glycopyrronium Bromide | 1.0 | 99.9 | 101.0 |
| Formoterol Fumarate | 0.5 | 93.8 | 100.3 |

| | | | |
|---|---|---|---|
| ⁽¹⁾ Active ingredient content compared to the theoretical amount expressed in percentage. | | | |

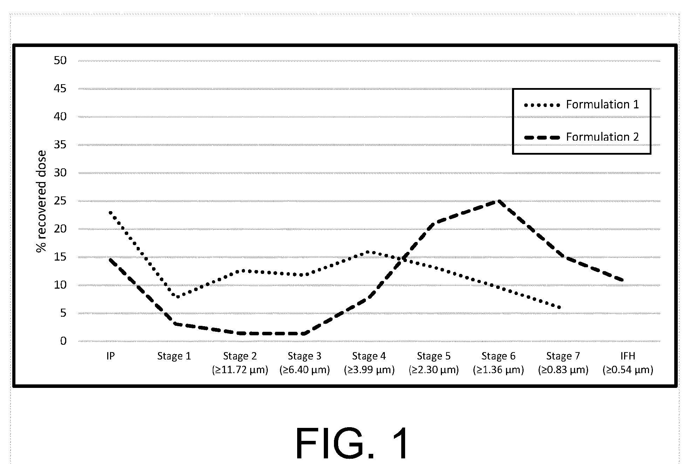

Also when comparing Formoterol in formulation 1 & 2, with an equivalent assay, Figure 1 shows that ethanol has an impact on the aerodynamic particle size distribution. In fact, ethanol allows for the particle size to be modified within the respirable range (extrafine particles or coarser ones).

The data shows that the higher the ethanol content, the lower is the particle size generated. Extrafine formulations are very desirable as they lead to deeper and more uniform distribution of the inhaled treatment in the lungs.

### Conclusions

In this study, it was demonstrated that beclometasone and formoterol solubilization are highly dependent on ethanol content. Beclometasone and Formoterol were not completely dissolved in 70% ethanol (Formulation 1) while all the active ingredients solubilized in 96% ethanol (Formulation 2).

In the NGI testing, Formulation 2 showed better aerodynamic properties than Formulation 1.

These results suggest therefore that a higher ethanol content could lead to more efficient formulations for inhaled therapies.

### Example 2 - Comparative of Trimbow^{®} formulation pMDI versus a soft mist inhaler

A study was performed to compare the aerodynamic particle size distribution and the emitted dose of a triple combination solution delivered with a soft mist inhaler device (Respimat^{®}) *versus* the commercialized product Trimbow^{®} pMDI (Triple combination product commercially available). The triple combination consists of the following active ingredients: beclomethasone dipropionate (BDP), glycopyrronium bromide (GB) and formoterol fumarate (FF) at the concentrations shown in Table 3.

Trimbow^{®} pMDI, a marketed product, was evaluated in this study as a product representative of triple combination products.

### Formulation and Inhaler System

Beclometasone dipropionate (BDP), glycopyrronium bromide (GB), formoterol fumarate (FF) and HCl 1N were mixed in 96% ethanol at 25°C for two hours. The solution was then filtrated and filled into cartridges compatible with a soft mist inhaler (Respimat^{®}).

Table 3 summarizes the products investigated in this study.

**Table 3. Formulations and devices investigated in this study.**

| **Device** | **Product Name** | **API/Excipients** | **Formula*** (µg/actuation) |
|---|---|---|---|
| Trimbow^{®} pMDI | Trimbow^{®} | Beclometasone Dipropionate | 87.4 µg |
| | | Glycopyrronium Bromide | 10.9 µg |
| | | Formoterol Fumarate | 5.2 µg |
| | | HCl 1N | 12.2 µg |
| | | HFA | 56645 µg |
| | | Ethanol | 9.6 µL |
| Soft Mist Inhaler | Triple combination with Soft Mist | Beclometasone Dipropionate | 87.4 µg |
| | | Glycopyrronium Bromide | 10.9 µg |
| | | Formoterol Fumarate | 5.2 µg |
| | | HCl 1N | 2.5 µg |
| | | Ethanol 96% | 11.1 µL |

| | | | |
|---|---|---|---|
| *Theoretical mass in µg per delivered dose. | | | |

### Aerodynamic particle size distribution (APSD)

The *in vitro* aerodynamic assessment was carried using a next generation impactor, NGI (Copley Scientific Ltd), equipped with a mouthpiece adapter for the insertion of the inhaler, an induction port and an internal filter holder (IFH) to capture the smaller particles. The product was tested as per procedure detailed in the European pharmacopoeia chapter 2.9.18 (apparatus E) and US Pharmacopeia chapter <601> (apparatus 6) for Soft Mist Inhalers.

The NGI was cooled to 5°C for at least 75 min. The cooling chamber was then opened and, after 30 minutes, the NGI was connected to a HCP5 vacuum pump (Copley Scientific Ltd). For each experiment, ten doses were discharged into the NGI at 30 L/min for 5 seconds each.

After the required actuations, the particles deposited on the different surfaces of the impactor were extracted using a suitable diluent (methanol) and with the help of a Gentle Rocker (Copley Scientific Ltd). Active ingredients were then quantified by HPLC, with the same methods used for the Example 1.

**Data analysis.** The *in vitro* aerodynamic assessment was carried using a next generation impactor, NGI (Copley Scientific Ltd), equipped with a mouthpiece adapter for the insertion of the inhaler, an induction port and an internal filter holder (IFH) to capture the smaller particles. The product was tested as per procedure detailed in the European pharmacopoeia chapter 2.9.18 (apparatus E) and US Pharmacopeia chapter <601> (apparatus 6) for Soft Mist Inhalers.

The NGI was cooled to 5°C for at least 75 min. The cooling chamber was then opened and, after 30 minutes, the NGI was connected to a HCP5 vacuum pump (Copley Scientific Ltd). For each experiment, ten doses were discharged into the NGI at 30 L/min for 5 seconds each.

After the required actuations, the particles deposited on the different surfaces of the impactor were extracted using a suitable diluent (methanol) and with the help of a Gentle Rocker (Copley Scientific Ltd). Active ingredients were then quantified by HPLC, with the same methods used for the Example 1.

**Data analysis.** The NGI results were plotted against the stage cut-off diameter as the mass recovered from the induction port to the filter. Fine particle fraction (FPF%), mass median aerodynamic diameter (MMAD), fine particle dose (FPD) were determined from the analysis of the NGI data.

Results are expressed as the mean value of two NGIs analysis.

### Delivered Dose

The delivered dose was tested according to USP <601> using a dose unit sampling apparatus (DUSA) operating at 28.3 L/min for 4 seconds - the inhalation volume did not exceed 2.0 L. Five doses per device were performed. Active pharmaceutical ingredients (APIs) deposited in the sampling apparatus were quantitatively recovered with a DUSA shaker (Copley Scientific Ltd) and methanol. APIs were, then, quantified by HPLC with the same methods described in Example 1.

Results are expressed as the average of 5 measurements.

### Results

Generally, drug particles from an inhaler will deposit in different parts of the lungs according to their size; coarser particles will deposit in the mouth and throat, middle size particles will deposit in the primary bronchi central airways while smaller particles will deposit in the terminal bronchi and alveoli. In this study, to determine the delivered dose and the detailed deposition rate of Trimbow^{®} pMDI and a soft mist inhaler, a dose unit sampling apparatus and a next generation impactor (NGI) were used, respectively.

Table 4 shows that one actuation of both devices released the same dose.

**Table 4. Triple combination Beclomethasone, Fomoterol and Glycopyrroniuum in a Soft Mist Inhaler (Respimat^{®}) and Trimbow^{®} pMDI. DUSA, Trimbow^{®} pMDI (1 actuation), Soft Mist Inhaler (1 actuation).**

| | **Soft Mist Inhaler (1 actuation)** | | **Trimbow^{®} pMDI (1 actuation)** | |
|---|---|---|---|---|
| **API** | **DUSA (µg)** | **RSD* (%)** | **DUSA (µg)** | **RSD (%)** |
| Beclometasone dipropionate | 97.1 | 2.9 | 98.4 | 3.2 |
| Glycopyrronium Bromide | 12.3 | 4.5 | 11.1 | 2.8 |
| Formoterol Fumarate | 6.1 | 2.8 | 5.7 | 2.3 |

| | | | | |
|---|---|---|---|---|
| *RSD: Relative Standard Deviation. | | | | |

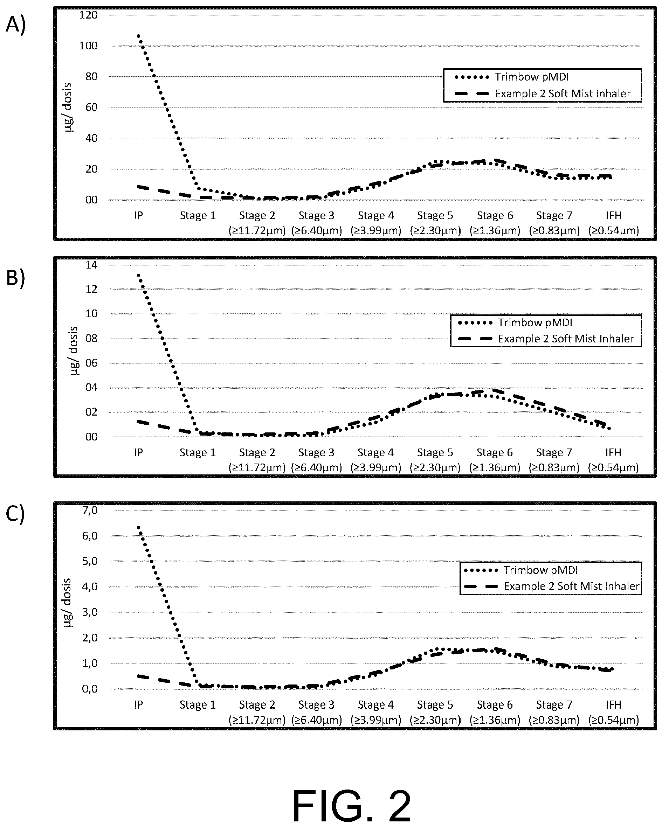

However, results from the NGI assay show that one single dose of the soft mist inhaler could simulate the same aerodynamic particle size distribution in terms of respirable particles - particles from stage 1 to internal filter holder (IFH) - as two doses of Trimbow^{®} pMDI. Also, the soft mist inhaler showed lower deposition in the induction port (IP) than Trimbow^{®} pMDI, which means a lower amount of coarser particles that are retained in the mouth and the throat (Figure 2).

MMAD values were around 1.2 µm, independently of the device used.

Taken together, these APSD results show that a soft mist inhaler offers, with 1 actuation containing the same quantity of drug per actuation, the same lung deposition and lower oropharyngeal deposition as Trimbow^{®} pMDI with 2 actuations, that are equivalent to 1 dose, as required per treatment. Thus, translating into a lower number of required actuations to achieve similar therapeutic effects and less drug being delivered to the oropharyngeal region. Due to possible side effects, deposition to the oropharyngeal area is not desired

**Table 5. APSD performance of the triple combination Beclomethasone, Fomoterol and Glycopyrronium in a Soft Mist Inhaler (Respimat^{®}) and the Trimbow^{®} pMDI. by NGI, Trimbow pMDI (2 actuations) vs Soft Mist Inhaler (1 actuation).**

| | Soft Mist Inhaler (1 actuation) | | | | Trimbow^{®} pMDI (2 actuations) | | | |
|---|---|---|---|---|---|---|---|---|
| API | MMAD (µm) | FPF (%) | FPD (µg) | IP (%) | MMAD (µm) | FPF (%) | FPD (µg) | IP (%) |
| Beclometasone dipropionate | 1.2 | 87.9 | 91.7 | 8.3 | 1.2 | 42.9 | 86.0 | 52.9 |
| Glycopyrronium Bromide | 1.3 | 86.7 | 12.0 | 9.0 | 1.2 | 43.4 | 10.6 | 54.2 |
| Formoterol Fumarate | 1.2 | 87.8 | 5.3 | 8.3 | 1.2 | 44.6 | 5.2 | 53.4 |

### Conclusions

Soft mist inhalers represent a novel approach to the delivery of inhaled drugs and overcome some of the limitations of DPIs and especially pMDIs.

In this study, though a soft mist inhaler and Trimbow^{®} pMDI showed similar delivered doses, the same lung deposition was observed with the soft mist inhaler with 1 actuation compared to 2 actuations of Trimbow^{®} pMDI - one single actuation with the soft mist inhaler simulates the lung deposition profile (and potentially same therapeutic effect) of two pMDI actuations. It is furthermore shown that similar distributions of particle sizes are achieved for each of the three active ingredients, despite their different physico-chemical properties. Hence, the idea underlying the present invention is applicable to different inhalable drug molecules.

### Example 3 - Comparative of Foster^{®} pMDI versus an equivalent formulation with a soft mist inhaler (Respimat^{®})

A study was performed to compare the aerodynamic particle size distribution of a double combination solution delivered with a soft mist inhaler device *versus* the commercially available product Foster^{®} pMDI. The double combination consists of the following active ingredients: beclomethasone dipropionate (BDP) and formoterol fumarate (FF) at the concentrations shown in Table 6.

Foster^{®} 100/6 µg pMDI, a marketed product, was evaluated in this study as product representative of double combination products.

### Formulation and Inhaler System

Beclometasone dipropionate (BDP), formoterol fumarate (FF) and HCl 1N were mixed in 96% ethanol at 25°C for two hours. The solution was then filtrated and filled into cartridges compatible with a Soft Mist Inhaler.

Table 6 summarizes the products investigated in this study.

**Table 6. Formulations and devices investigated in this study.**

| Device | Product Name | API/Excipients | Formula* (µg/actuation) |
|---|---|---|---|
| Foster^{®} pMDI | Foster^{®} | Beclometasone Dipropionate | 84.6 µg |
| | | Formoterol Fumarate | 5.0 µg |
| | | HCl 1N | 11.9 µg |
| | | HFA | 55034 µg |
| | | Ethanol | 9.3 µL |
| Soft Mist Inhaler | Double combination in Soft Mist Inhaler | Beclometasone Dipropionate | 84.6 µg |
| | | Formoterol Fumarate | 5.0 µg |
| | | HCl 1N | 2 µg |
| | | Etanol 96% | 11.1 µL |

| | | | |
|---|---|---|---|
| *Theoretical mass in µg per delivered dose. | | | |

### Aerodynamic particle size distribution (APSD)

The *in vitro* aerodynamic assessment was carried using a next generation impactor, NGI (Copley Scientific Ltd), equipped with a mouthpiece adapter for the insertion of the inhaler, an induction port and an internal filter holder (IFH) to capture the smaller particles. The product was tested as per procedure detailed in the European and US Pharmacopeia for Soft Mist Inhalers and pMDIs.

The NGI was cooled to 5°C for at least 75 min. The cooling chamber was then opened and, after 30 minutes, the NGI was connected to a HCP5 vacuum pump (Copley Scientific Ltd). For each experiment, ten doses were discharged into the NGI at 30 L/min for 5 seconds each.

After the required actuations, the particles deposited on the different surfaces of the impactor were extracted using a suitable diluent (methanol) and with the help of a Gentle Rocker (Copley Scientific Ltd). Active ingredients were then quantified by HPLC, with the same methods used for the Example 1.

**Data analysis.** The *in vitro* aerodynamic assessment was carried using a next generation impactor, NGI (Copley Scientific Ltd), equipped with a mouthpiece adapter for the insertion of the inhaler, an induction port and an internal filter holder (IFH) to capture the smaller particles. The product was tested as per procedure detailed in the European pharmacopoeia chapter 2.9.18 (apparatus E) and US Pharmacopeia chapter <601> (apparatus 6) for Soft Mist Inhalers.

The NGI was cooled to 5°C for at least 75 min. The cooling chamber was then opened and, after 30 minutes, the NGI was connected to a HCP5 vacuum pump (Copley Scientific Ltd). For each experiment, ten doses were discharged into the NGI at 30 L/min for 5 seconds each.

After the required actuations, the particles deposited on the different surfaces of the impactor were extracted using a suitable diluent (methanol) and with the help of a Gentle Rocker (Copley Scientific Ltd). Active ingredients were then quantified by HPLC, with the same methods used for the Example 1.

**Data analysis.** The NGI was plotted against the stage cut-off diameter as the mass recovered from the induction port to the filter. Fine particle fraction (FPF%) and mass median aerodynamic diameter (MMAD) were determined from the analysis of the NGI data.

Results are expressed as the mean value of two NGIs analysis.

### Results

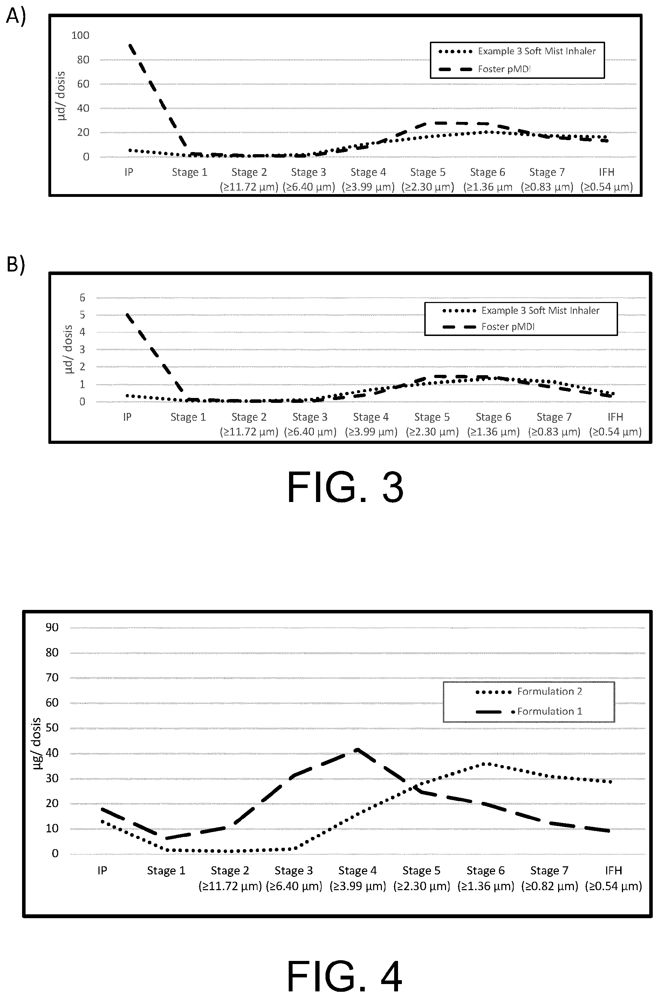

The deposition rate of Foster^{®} pMDI and Soft Mist Inhaler was determined by a next generation impactor. Results showed that one single dose of Soft Mist Inhaler double the FPF values of one actuation of Foster^{®} pMDI. MMAD values were around 1.2 µm, independently the device used (Table 7 and Figure 3).

**Table 7. APSD performance of the Soft Mist Inhaler and the Foster^{®} pMDI. NGI, Foster pMDI (2 actuation) vs Soft Mist Inhaler (1 actuation).**

| | **Soft Mist Inhaler (1 actuation)** | | **Trimbow^{®} pMDI (2 actuations)** | |
|---|---|---|---|---|
| **API** | **MMAD (µm)** | **FPF (%)** | **MMAD (µm)** | **FPF (%)** |
| Beclometasone dipropionate | 1.1 | 91.3 | 1.2 | 49.4 |
| Formoterol Fumarate | 1.2 | 90.4 | 1.2 | 45.8 |

### Conclusions

Results from this study show that one single actuation with Soft Mist Inhaler could simulate the lung deposition rate (and potentially same therapeutic effect) of two pMDI actuations.

Taken together, these APSD results were in agreement with those obtained in example 2 and show the potential of Soft Mist Inhaler not only for triple combination therapies but also for double combination treatments.

### Example 4 - Impact of ethanol in the aerodynamic particle size distribution of a double combination therapy

A study was performed to evaluate the impact of ethanol in the aerodynamic particle size distribution of a double combination solution delivered with a soft mist inhaler device (Respimat^{®}). The double combination consists of the following active ingredients: budesonide (BU) and formoterol fumarate (FF) at the concentrations shown in Table 8.

### Formulation and Inhaler System

Budesonide (BU) and formoterol fumarate (FF) were mixed in 70% ethanol (Formulation 1) and 96% ethanol (Formulation 2) at 25°C for two hours protected from evaporation. Both formulations were then filtrated, and filled into cartridges compatible with soft mist inhaler (Respimat^{®}).

Table 8 summarizes the products investigated in this study.

**Table 8. Formulations investigated in this study.**

| **Solution** | **API/Excipients** | **Formula*** (µg/actuation) |
|---|---|---|
| Formulation 1 | Budesonide | 160.0 µg |
| | Formoterol Fumarate | 4.5 µg |
| | Water for Irrigation | 3.32 µL |
| | Ethanol absolute | 7.74 µL |
| Formulation 2 | Budesonide | 160.0 µg |
| | Formoterol Fumarate | 4.5 µg |
| | Ethanol 96% | 11.05 µL |

| | | |
|---|---|---|
| *Theoretical mass in µg per delivered dose. | | |

### HPLC Analytical methods

One Reverse Phase HPLC- UV analytical method was used for the determination of both active ingredients.

An Isocratic (1.0mL/min), HPLC-UV detection (Budesonide wavelength: 240nm, Formoterol Fumarate wavelength: 214nm) was employed with a Mobile Phase composed of 50/50 v/v ACN/0.1 % Formic Acid with a C18 column (Hypersil BDS, 250 x 4.6 mm, 5 µm). External Formoterol Fumarate and Budesonide standards prepared in the method diluent (75/25 v/v MeOH/water) were used to quantify the amount of active ingredient present in the sample prepared in the same diluent using the response factor of Standard and Sample solutions. Typical System Suitability Criteria applies as per USP <621> requirements.

### Aerodynamic particle size distribution (APSD)

For the APSD evaluation Formulation 1 and 2 were filled into cartridges compatible with a soft mist inhaler (Respimat^{®}). The *in vitro* aerodynamic particle size distribution of both formulations was evaluated using an impactor, the next generation impactor, NGI (Copley Scientific Ltd) equipped with a mouthpiece adapter for the insertion of the inhaler, an induction port and an internal filter holder (IFH) to capture the smaller particles. The product was tested as per procedure detailed in the European pharmacopoeia chapter 2.9.18 (apparatus E) and US Pharmacopeia chapter <601> (apparatus 6) for Soft Mist Inhalers.

The NGI was cooled to 5°C for at least 75 min. The cooling chamber was then opened and, after 30 minutes, the NGI was connected to a HCP5 vacuum pump (Copley Scientific Ltd). For each experiment, ten doses were discharged per NGI actuated at 28.3 L/min for 5 seconds each.

After the required actuations, the particles deposited on the different surfaces of the impactor were extracted using a suitable diluent (75/25 v/v MeOH/water) and with the help of a Gentle Rocker (Copley Scientific Ltd). Active ingredients were then quantified by HPLC as described above.

**Data analysis.** The NGI results were plotted against the stage cut-off diameter as the mass recovered from the induction port to the filter. Fine particle fraction (FPF%), mass median aerodynamic diameter (MMAD), fine particle dose (FPD) were determined from the analysis of the NGI data.

Results are expressed as the mean value of two NGIs analysis.

### Results

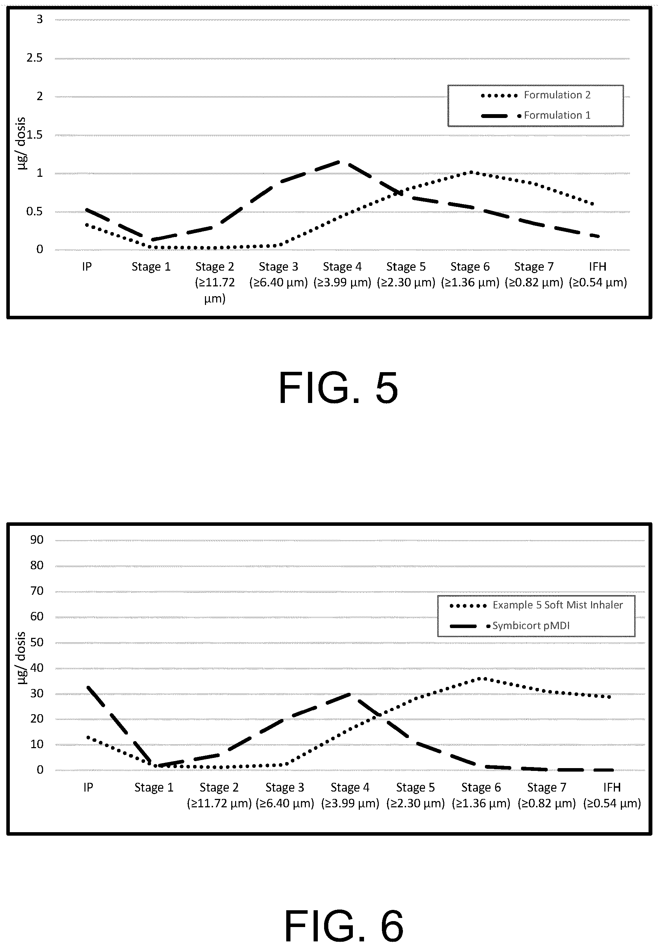

The deposition rate of Formulation 1 and Formulation 2 was determined by a next generation impactor. Results showed that ethanol has an impact on the aerodynamic particle size distribution (Table 9 and Figure 4 and 5). In fact, a higher ethanol content led to an increase in FPF values and a lower particle size distribution. Extrafine particle size distribution are very desirable as they lead to more efficient treatments in inhaled therapies.

**Table 9. APSD performance of Formulation 1 and Formulation 2 delivered with a Soft Mist Inhaler.**

| | **Formulation 1 (70% EtOH)** | | | **Formulation 2 (96% EtOH)** | | |
|---|---|---|---|---|---|---|
| **API** | **MMAD (µm)** | **FPF (%)** | **FPD (µg)** | **MMAD (µm)** | **FPF (%)** | **FPF (µg)** |
| Budesonide | 2.6 | 68.5 | 119.1 | 1.1 | 89.2 | 141.0 |
| Formoterol Fumarate | 2.7 | 68.4 | 3.3 | 1.1 | 89.7 | 3.7 |

### Conclusions

In inhalation therapies, extrafine formulations lead to deeper and more uniform lung distribution. In this study, it was demonstrated that a higher ethanol content provides a lower particle size distribution and therefore gives better aerodynamic properties to the formulation. Results are in line with Example 1 and confirm the potential that ethanol has to generate more efficient formulations for inhaled therapies.

### Example 5 - Comparative of Symbicort^{®} pMDI versus an equivalent ethanolic formulation delivered with a soft mist inhaler (Respimat^{®})

A study was performed to compare the aerodynamic particle distribution of a double combination ethanolic solution delivered with a soft mist inhaler device (Respimat^{®}) versus the commercialized product Symbicort^{®} pMDI (double combination product commercially available). The double combination consists of the following active ingredients: Budesonide (BU) and formoterol fumarate (FF) at the concentrations shown in Table 10.

Symbicort^{®} 160/4.5 µg pMDI, a marketed product, was evaluated in this study as product representative of double combination products with no ethanol in their formula.

### Formulation and Inhaler System

Budesonide (BU) and formoterol fumarate (FF) were mixed in 96% ethanol at 25°C for two hours protected from evaporation. The solution was then filtrated and filled into cartridges compatible with soft mist inhaler (Respimat^{®}).

Table 10 summarizes the products investigated in this study.

**Table 10. Formulations investigated in this study.**

| **Device** | **API/Excipients** | **Formula*** (µg/actuation) |
|---|---|---|
| Symbicort^{®} pMDI | Budesonide | 160.0 µg |
| | Formoterol Fumarate | 4.5 µg |
| | HFA227 | q.s. |
| | Povidone | q.s. |
| | Macrogol | q.s. |
| Soft mist inhaler | Budesonide | 160.0 µg |
| | Formoterol Fumarate | 4.5 µg |
| | Ethanol 96% | 11.05 µL |

| | | |
|---|---|---|
| *Theoretical mass in µg per delivered dose. | | |

### Aerodynamic particle size distribution (APSD)

The *in vitro* aerodynamic particle size distribution of both formulations was evaluated using an impactor, the next generation impactor, NGI (Copley Scientific Ltd) equipped with a mouthpiece adapter for the insertion of the inhaler, an induction port and an internal filter holder (IFH) to capture the smaller particles. The product was tested as per procedure detailed in the European and US Pharmacopoeia for Soft Mist Inhalers and pMDIs.

The NGI was cooled to 5°C for at least 75 min. The cooling chamber was then opened and, after 30 minutes, the NGI was connected to a HCP5 vacuum pump (Copley Scientific Ltd). For each experiment, ten doses were discharged per NGI actuated at 28.3 L/min for 5 seconds each.

After the required actuations, the particles deposited on the different surfaces of the impactor were extracted using a suitable diluent (75/25 v/v MeOH/water) and with the help of a Gentle Rocker (Copley Scientific Ltd). Active ingredients were then quantified by HPLC, with the same methods described in example 4.

**Data analysis.** The NGI was plotted against the stage cut-off diameter as the mass recovered from the induction port to the filter. Fine particle fraction (FPF%), and mass median aerodynamic diameter (MMAD) were determined from the analysis of the NGI data.

Results are expressed as the mean value of two NGIs analysis.

### Results

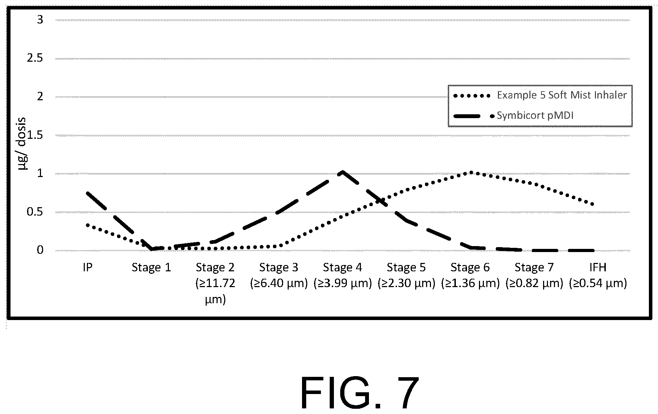

The deposition rate of Symbicort^{®} pMDI and Soft Mist Inhaler was determined by a next generation impactor. Results showed that the Soft Mist Inhaler composition has an impact on the aerodynamic particle size distribution (Table 11 and Figure 6 and 7). The Soft Mist Inhaler composition led to a smaller particle size distribution achieving MMAD values of around 1.1 µm. This is very positive since small particle size distribution leads to deeper and more uniform lung distribution.

Moreover, ethanolic solutions delivered with a soft mist inhaler led FPF values of around 1.5-fold higher than the pMDI product.

**Table 11. APSD performance of Symbicort^{®} pMDI and the Soft Mist Inhaler. NGI, Symbicort^{®} pMDI (1 actuation) vs Soft Mist Inhaler (1 actuation).**

| | **Symbicort^{®} pMDI (1 actuation)** | | **Soft Mist Inhaler (1 actuation)** | |
|---|---|---|---|---|
| **API** | **MMAD (µm)** | **FPF (%)** | **MMAD (µm)** | **FPF (%)** |
| Budesonide | 3.7 | 49.7 | 1.1 | 89.2 |
| Formoterol Fumarate | 3.3 | 58.7 | 1.1 | 89.7 |

### Conclusions

In this study, it was demonstrated that the Soft Mist Inhaler composition leads to smaller particle size distributions. Thus, translating into a deeper and more efficient lung distribution. Moreover, the solution delivered with the Soft Mist Inhaler showed higher FPF values, resulting in lower doses required to achieve similar therapeutic effects than the pMDI and potentially lower side effects due to less drug being delivered to the oropharyngeal region.

## Claims

1. A pharmaceutical solution composition for delivery to the pulmonary system via a soft mist inhaler comprising:
(a) an inhaled corticosteroid (ICS),
(b) a long-acting beta-agonist (LABA), and
(c) optionally a long-acting muscarinic antagonist (LAMA), dissolved in a pharmaceutically acceptable solvent, wherein the solvent comprises ethanol in an amount of 75 to 100% v/v.

2. The pharmaceutical solution composition according to claim 1, wherein said solvent comprises ethanol in an amount of 80-99% v/v.

3. The pharmaceutical solution composition according to claim 1 or 2, wherein said solvent comprises ethanol in an amount of 90-97% v/v.

4. The pharmaceutical solution composition according to any of claims 1 to 3, wherein the inhaled corticosteroid is selected from the group consisting of beclomethasone dipropionate, budesonide, ciclesonide, fluticasone propionate; fluticasone furoate, and mometasone; the long-acting beta-agonist is selected from the group consisting of formoterol fumarate, salmeterol, indacaterol, vilanterol, and odolaterol; and the long-acting muscarinic antagonist is selected from the group consisting of glycopyrronium bromide, umeclidinium, aclidinium, ipratropium, tiotropium, and oxitropium.

5. The pharmaceutical solution composition according to claim 4, wherein the inhaled corticosteroid is beclomethasone dipropionate, the long-acting beta-agonist is formoterol fumarate, and the long-acting muscarinic antagonist is glycopyrronium bromide.

6. The pharmaceutical solution composition according to any of claims 1 to 5, wherein the weight ratio by percentage (% w/w) of ICS to LABA is 30-100% to 0.5-50%.

7. The pharmaceutical solution composition according to any of claims 1 to 5, wherein the weight ratio by percentage (% w/w/w) of the ICS, LABA and LAMA is 30-100%, 0.5-50% and 0.5-50%, respectively.

8. The pharmaceutical solution composition according to any of claims 1 to 7, wherein the solution further comprises one or more pharmacologically acceptable acids and/or buffers for adjusting the pH.

9. The pharmaceutical solution composition according to any of claims 1 to 8, wherein said composition does not contain any propellant, such as a hydrofluorocarbon, e.g norflurane.

10. The pharmaceutical solution composition according to any of claims 1 to 9, wherein said composition does not contain any preservative, such as benzalkonium chloride.

11. A container comprising a pharmaceutical solution composition according to any of claims 1 to 10, wherein said container is in a form suitable for use with a soft mist inhaler.

12. A kit comprising a container according to claim 11 and a soft mist inhaler.

13. The pharmaceutical solution composition according to claims 1 to 10 for use in the prevention and/or treatment of an inflammatory and/or obstructive airways disease.

14. The pharmaceutical solution composition for use in the prevention and/or treatment of an inflammatory and/or obstructive airways disease according to claim 13, wherein said disease is asthma or COPD.

## Patentansprüche

1. Pharmazeutische Lösungszusammensetzung zur Abgabe an das Lungensystem über ein Feuchtinhalationsgerät, umfassend:
(a) ein inhaliertes Kortikosteroid (ICS),
(b) einen langwirksamen Beta-Agonisten (LABA), und
(c) optional einen langwirksamen Muskarin-Antagonisten (LAMA), gelöst in einem pharmazeutisch verträglichen Lösungsmittel, wobei das Lösungsmittel Ethanol in einer Menge von 75 bis 100 Gew.-% umfasst.

2. Pharmazeutische Lösungszusammensetzung nach Anspruch 1, wobei das Lösungsmittel Ethanol in einer Menge von 80-99 Gew.-% umfasst.

3. Pharmazeutische Lösungszusammensetzung nach Anspruch 1 oder 2, wobei das Lösungsmittel Ethanol in einer Menge von 90-97 Gew.-% umfasst.

4. Pharmazeutische Lösungszusammensetzung nach einem der Ansprüche 1 bis 3, wobei das inhalierte Kortikosteroid aus der Gruppe ausgewählt ist, bestehend aus Beclomethasondipropionat, Budesonid, Ciclesonid, Fluticasonpropionat; Fluticasonfuroat, und Mometason; der langwirksame Beta-Agonist aus der Gruppe ausgewählt ist, bestehend aus Formoterolfumarat, Salmeterol, Indacaterol, Vilanterol, und Odolaterol; und der langwirksame Muskarin-Antagonist aus der Gruppe ausgewählt ist, bestehend aus Glycopyrroniumbromid, Umeclidinium, Aclidinium, Ipratropium, Tiotropium und Oxitropium.

5. Pharmazeutische Lösungszusammensetzung nach Anspruch 4, wobei das inhalierte Kortikosteroid Beclomethasondipropionat, der langwirksame Beta-Agonist Formoterolfumarat und der langwirksame Muskarinantagonist Glycopyrroniumbromid ist.

6. Pharmazeutische Lösungszusammensetzung nach einem der Ansprüche 1 bis 5, wobei das Gewichtsverhältnis in Prozent (Gew.-%) von ICS zu LABA 30-100 % bis 0,5-50% beträgt.

7. Pharmazeutische Lösungszusammensetzung nach einem der Ansprüche 1 bis 5, wobei das Gewichtsverhältnis in Prozent (Gew.-%) des ICS, LABA und LAMA jeweils 30-100%, 0,5-50% und 0,5-50% beträgt.

8. Pharmazeutische Lösungszusammensetzung nach einem der Ansprüche 1 bis 7, wobei die Lösung weiter einen oder mehrere pharmazeutisch verträgliche Säuren und/oder Puffer zum Anpassen des pH-Werts umfasst.

9. Pharmazeutische Lösungszusammensetzung nach einem der Ansprüche 1 bis 8, wobei die Zusammensetzung kein Treibmittel, wie etwa einen Fluorkohlenwasserstoff, z. B. Norfluran, enthält.

10. Pharmazeutische Lösungszusammensetzung nach einem der Ansprüche 1 bis 9, wobei die Zusammensetzung kein Konservierungsmittel, wie etwa Benzalkoniumchlorid, enthält.

11. Behälter, der eine pharmazeutische Lösungszusammensetzung nach einem der Ansprüche 1 bis 10 umfasst, wobei der Behälter in einer Form ist, die zur Verwendung mit einem Feuchtinhalationsgerät geeignet ist.

12. Kit, das einen Behälter nach Anspruch 11 und ein Feuchtinhalationsgerät umfasst.

13. Pharmazeutische Lösungszusammensetzung nach den Ansprüchen 1 bis 10 zur Verwendung bei der Vorbeugung und/oder Behandlung einer entzündlichen und/oder obstruktiven Atemwegserkrankung.

14. Pharmazeutische Lösungszusammensetzung zur Verwendung bei der Vorbeugung und/oder Behandlung einer entzündlichen und/oder obstruktiven Atemwegserkrankung nach Anspruch 13, wobei die Erkrankung Asthma oder COPD ist.

## Revendications

1. Composition de solution pharmaceutique destinée à être administrée au système pulmonaire au moyen d'un inhalateur à brume douce, comprenant :
(a) un corticostéroïde inhalé (CSI),
(b) un bêta-agoniste à action prolongée (LABA), et
(c) facultativement, un antagoniste muscarinique à action prolongée (LAMA), dissous dans un solvant pharmaceutiquement acceptable, dans laquelle le solvant comprend de l'éthanol en une quantité de 75 à 100 % v/v.

2. Composition de solution pharmaceutique selon la revendication 1, dans laquelle ledit solvant comprend de l'éthanol en une quantité de 80-99 % v/v.

3. Composition de solution pharmaceutique selon la revendication 1 ou 2, dans laquelle ledit solvant comprend de l'éthanol en une quantité de 90-97 % v/v.

4. Composition de solution pharmaceutique selon l'une quelconque des revendications 1 à 3, dans laquelle le corticostéroïde inhalé est choisi dans le groupe constitué par le dipropionate de béclométhasone, le budésonide, le ciclésonide, le propionate de fluticasone ; le furoate de fluticasone et la mométasone ; le bêta-agoniste à action prolongée est choisi dans le groupe constitué par le fumarate de formotérol, le salmétérol, l'indacatérol, le vilanterol et l'odolatérol ; et l'antagoniste muscarinique à action prolongée est choisi dans le groupe constitué par le bromure de glycopyrronium, l'uméclidinium, l'aclidinium, l'ipratropium, le tiotropium et l'oxitropium.

5. Composition de solution pharmaceutique selon la revendication 4, dans laquelle le corticostéroïde inhalé est le dipropionate de béclométhasone, le bêta-agoniste à action prolongée est le fumarate de formotérol et l'antagoniste muscarinique à action prolongée est le bromure de glycopyrronium.

6. Composition de solution pharmaceutique selon l'une quelconque des revendications 1 à 5, dans laquelle le rapport pondéral en pourcentage (% p/p) de l'ICS à LABA est 30-100 % à 0,5-50 %.

7. Composition de solution pharmaceutique selon l'une quelconque des revendications 1 à 5, dans laquelle le rapport pondéral en pourcentage (% p/p/p) de l'ICS, du LABA et de LAMA est 30-100 %, 0,5-50 % et 0,5-50 %, respectivement.

8. Composition de solution pharmaceutique selon l'une quelconque des revendications 1 à 7, dans laquelle la solution comprend en outre un ou plusieurs acides et/ou tampons pharmacologiquement acceptables pour ajuster le pH.

9. Composition de solution pharmaceutique selon l'une quelconque des revendications 1 à 8, dans laquelle ladite composition ne contient aucun propulseur, tel qu'un hydrofluorocarbure, par exemple le norflurane.

10. Composition de solution pharmaceutique selon l'une quelconque des revendications 1 à 9, dans laquelle ladite composition ne contient aucun conservateur, tel que le chlorure de benzalkonium.

11. Contenant comprenant une composition de solution pharmaceutique selon l'une quelconque des revendications 1 à 10, dans lequel ledit contenant est sous une forme appropriée à une utilisation avec un inhalateur à brume douce.

12. Kit comprenant un contenant selon la revendication 11 et un inhalateur à brume douce.

13. Composition de solution pharmaceutique selon les revendications 1 à 10 destinée à être utilisée dans la prévention et/ou le traitement d'une maladie inflammatoire et/ou d'une maladie obstructive des voies aériennes.

14. Composition de solution pharmaceutique destinée à être utilisée dans la prévention et/ou le traitement d'une maladie inflammatoire et/ou d'une maladie obstructive des voies aériennes selon la revendication 13, dans laquelle ladite maladie est l'asthme ou la BPCO.
